# EUROPEAN PATENT SPECIFICATION

(11) **EP 2 791 680 B1**
(45) Date of publication and mention of the grant of the patent: **09.12.2015**
(21) Application number: 12818607.9
(22) Date of filing: 12.12.2012
(51) Int. Cl.: G01N 33/569

(54) **ASSAY FOR INFLUENZA VIRUS HEMAGGLUTININS**
ASSAY FÜR INFLUENZAVIRUS-HÄMAGGLUTININE
ANALYSE POUR HÉMAGGLUTININES DU VIRUS DE LA GRIPPE

(30) Priority: 12.12.2011 US 201161569597 P
(43) Date of publication of application: 22.10.2014
(73) Proprietor: Novartis AG, 4056 Basel (CH)
(72) Inventor: DORMITZER, Philip, Cambridge, MA 02139 (US); ANDREWS, William, Cambridge, MA 02139 (US); RINELLA, Paola, 53100 Siena (IT); ROSA, Domenico, 53100 Siena (IT); PALLADINO, Giuseppe, Cambridge, MA 02139 (US)
(74) Representative: Marshall, Cameron John
(86) International application number: PCT/IB2012/057235
(87) International publication number: WO 2013/088367

(56) References cited:
- WO-A1-2010/136896
- YUICHIRO SATO ET AL: "High mannose-specific lectin (KAA-2) from the red algapotently inhibits influenza virus infection in a strain-independent manner", BIOCHEMICAL AND BIOPHYSICAL RESEARCH COMMUNICATIONS, vol. 405, no. 2, 8 January 2011 (2011-01-08), pages 291-296, XP028361653, ISSN: 0006-291X, DOI: 10.1016/J.BBRC.2011.01.031 [retrieved on 2011-01-08]
- KIM Y K ET AL: "Evaluation of new hemagglutinin-based rapid antigen test for influenza A pandemic (H1N1) 2009", JOURNAL OF CLINICAL VIROLOGY, ELSEVIER, AMSTERDAM, NL, vol. 49, no. 1, 1 September 2010 (2010-09-01), pages 69-72, XP027212183, ISSN: 1386-6532 [retrieved on 2010-08-12]
- HUAI-LONG XU ET AL: "Molecular modeling, docking and dynamics simulations of GNA-related lectins for potential prevention of influenza virus (H1N1)", JOURNAL OF MOLECULAR MODELING, SPRINGER VERLAG, DE, vol. 18, no. 1, 29 March 2011 (2011-03-29) , pages 27-37, XP019994702, ISSN: 0948-5023, DOI: 10.1007/S00894-011-1022-7

### SEQUENCE LISTING

<110> Novartis AG
<120> Assays for influenza virus hemagglutinins
<130> P060225WO
<141> 2012-12-12
<150> US 61/569,597
   <151> 2011-12-12
<160> 6
<170> Seqwin 2010, version 1.0
<210> 1
   <211> 157
   <212> PRT
   <213> Galanthus nivalis
<400> 1
   Met Ala Lys Ala Ser Leu Leu Ile Leu Ala Thr Ile Phe Leu Gly Val
   1               5                   10                  15
   Ile Thr Pro Ser Cys Leu Ser Glu Asn Ile Leu Tyr Ser Gly Glu Thr
               20                  25                  30
   Leu Pro Thr Gly Gly Phe Leu Ser Ser Gly Ser Phe Val Phe Ile Met
           35                  40                  45
   Gln Glu Asp Cys Asn Leu Val Leu Tyr Asn Val Asp Lys Pro Ile Trp
       50                  55                  60
   Ala Thr Asn Thr Gly Gly Leu Ser Ser Asp Cys Ser Leu Ser Met Gln
   65                  70                  75                  80
   Asn Asp Gly Asn Leu Val Val Phe Thr Pro Ser Asn Lys Pro Ile Trp
                   85                  90                  95
   Ala Ser Asn Thr Asp Gly Gln Asn Gly Asn Tyr Val Cys Ile Leu Gln
               100                 105                 110
   Lys Asp Arg Asn Val Val Ile Tyr Gly Thr Asn Arg Trp Ala Thr Gly
           115                 120                 125
   Thr Tyr Thr Gly Ala Val Gly Ile Pro Glu Ser Pro Pro Ser Glu Lys
       130                 135                 140
   Tyr Pro Ser Ala Gly Lys Ile Lys Leu Val Thr Ala Lys
   145                 150                 155
<210> 2
   <211> 157
   <212> PRT
   <213> Galanthus nivalis
<400> 2
   Met Ala Lys Ala Ser Leu Leu Ile Leu Thr Thr Ile Phe Leu Gly Val
   1               5                   10                  15
   Ile Thr Pro Ser Cys Leu Ser Glu Asn Ile Leu Tyr Ser Gly Glu Thr
               20                  25                  30
   Leu Pro Thr Gly Gly Ser Leu Thr Ser Gly Ser Phe Val Phe Ile Met
           35                  40                  45
   Gln Gln Asp Cys Asn Leu Val Leu Tyr Asn Val Asp Lys Pro Ile Trp
           50                  55                  60
   Ala Thr Asn Thr Gly Gly Leu Ser Ser Asp Cys Ser Ile Ser Leu Gln
   65                  70                  75                  80
   Thr Asp Gly Asp Leu Val Val Tyr Thr Pro Ser Asn Lys Pro Ile Trp
                   85                  90                  95
   Ala Ser Asn Thr Asp Gly Gln Asn Gly Asn Tyr Val Cys Ile Leu Gln
               100                 105                 110
   Lys Asp Arg Asn Val Val Ile Tyr Gly Thr Asn Arg Trp Ala Thr Gly
           115                 120                 125
   Thr Tyr Thr Gly Ala Val Gly Ile Pro Glu Ser Pro Pro Ser Glu Lys
       130                 135                 140
   Tyr Pro Thr Ala Gly Met Ile Lys Leu Val Thr Ala Lys
   145                 150                 155
<210> 3
   <211> 157
   <212> PRT
   <213> Galanthus nivalis
<400> 3
   Met Ala Lys Ala Ser Leu Leu Ile Leu Ala Thr Ile Phe Leu Gly Val
   1               5                   10                  15
   Ile Thr Pro Ala Cys Leu Ser Glu Asn Ile Leu Tyr Ser Gly Glu Thr
               20                  25                  30
   Leu Pro Thr Gly Gly Phe Leu Thr Ser Gly Ser Phe Val Phe Ile Met
           35                  40                  45
   Gln Glu Asp Cys Asn Leu Val Leu Tyr Asn Val Asp Lys Pro Ile Trp
       50                  55                  60
   Ala Thr Asn Thr Gly Gly Leu Ser Ser Asp Cys Ser Leu Ser Met Gln
   65                  70                  75                  80
   Thr Asp Gly Asn Leu Val Val Tyr Thr Pro Ser Asp Lys Pro Ile Trp
                   85                  90                  95
   Ala Ser Asp Thr Asp Gly Gln Asn Gly Asn Tyr Val Cys Ile His Gln
               100                 105                 110
   Lys Asp Arg Asn Val Val Ile Tyr Gly Thr Asn Arg Trp Ala Thr Gly
           115                 120                 125
   Thr His Thr Gly Ala Val Gly Ile Pro Ala Ser Pro Pro Ser Glu Lys
       130                 135                 140
   Tyr Pro Thr Ala Gly Met Ile Lys Leu Val Thr Ala Lys
   145                 150                 155
<210> 4
   <211> 157
   <212> PRT
   <213> Galanthus nivalis
<400> 4
   Met Ala Lys Ala Ser Leu Leu Ile Leu Ala Thr Ile Phe Leu Gly Val
   1               5                   10                  15
   Ile Thr Pro Ser Cys Leu Ser Glu Asn Ile Leu Tyr Ser Gly Glu Thr
               20                  25                  30
   Leu Pro Thr Gly Gly Phe Leu Thr Ser Gly Ser Phe Val Phe Ile Met
           35                  40                  45
   Gln Gln Asp Cys Asn Leu Val Leu Tyr Asn Ile Asp Lys Pro Ile Trp
       50                  55                  60
   Ala Thr Asn Thr Cys Gly Leu Ser Ser Asp Cys Ser Leu Ser Met Gln
   65                  70                  75                  80
   Thr Asp Gly Asn Leu Val Val Tyr Thr Pro Ser Asn Lys Pro Ile Trp
                   85                  90                  95
   Ala Ser Asn Thr Asp Gly Gln Asn Gly Asn Tyr Val Cys Ile Leu Gln
               100                 105                 110
   Lys Asp Arg Asn Val Val Ile Tyr Gly Thr Asn Arg Trp Ala Thr Gly
           115                 120                 125
   Thr His Thr Gly Ala Val Gly Ile Leu Ala Ser Pro Pro Ser Glu Lys
       130                 135                 140
   Tyr Pro Thr Ala Gly Met Ile Lys Leu Val Thr Ala Lys
   145                 150                 155
<210> 5
   <211> 157
   <212> PRT
   <213> Galanthus nivalis
<400> 5
   Met Ala Lys Ala Ser Leu Leu Ile Leu Ala Thr Ile Phe Leu Gly Val
   1               5                   10                  15
   Ile Thr Pro Ala Cys Leu Ser Glu Asn Ile Leu Tyr Ser Gly Glu Thr
               20                  25                  30
   Leu Pro Thr Gly Gly Phe Leu Thr Ser Gly Ser Phe Val Phe Ile Met
           35                  40                  45
   Gln Gln Asp Cys Asn Leu Val Leu Tyr Asn Ile Asp Lys Pro Ile Trp
       50                  55                  60
   Ala Thr Asn Thr Gly Gly Leu Ser Ser Asp Cys Ser Leu Ser Met Gln
   65                  70                  75                  80
   Thr Asp Gly Asn Leu Val Val Tyr Asn Pro Ser Asn Lys Pro Ile Trp
                   85                  90                  95
   Ala Ser Asn Thr Asp Gly Gln Asn Gly Asn Tyr Val Cys Ile Leu Gln
               100                 105                 110
   Lys Asp Arg Asn Val Val Ile Tyr Gly Thr Asn Arg Trp Ala Thr Gly
           115                 120                 125
   Thr His Thr Gly Ala Val Gly Ile Leu Ala Ser Pro Pro Ser Glu Lys
       130                 135                 140
   Tyr Pro Thr Ala Gly Met Ile Lys Leu Val Thr Ala Lys
   145                 150                 155
<210> 6
   <211> 160
   <212> PRT
   <213> Galanthus nivalis
<400> 6
   Met Ala Lys Ala Ser Leu Leu Ile Leu Ala Ala Ile Phe Leu Gly Val
   1               5                   10                  15
   Ile Thr Pro Ser Ser Pro Ser Cys Leu Gly Asp Asn Ile Leu Tyr Ser
                   20                  25                  30
   Gly Glu Thr Leu Ser Thr Gly Glu Phe Leu Asn Tyr Gly Gly Phe Val
           35                  40                  45
   Phe Ile Met Gln Glu Asp Cys Asn Leu Val Leu Tyr Asp Val Asp Lys
       50                  55                  60
   Pro Ile Trp Ala Thr Asn Thr Gly Gly Leu Ser Arg Ser Cys Phe Leu
   65                  70                  75                  80
   Ser Met Gln Thr Asp Gly Asn Leu Val Val Tyr Thr Pro Ser Asn Lys
                   85                  90                  95
   Pro Ile Trp Ala Ser Asn Thr Gly Gly Gln Asn Gly Asn Tyr Val Cys
               100                 105                 110
   Ile Leu Gln Lys Asp Arg Asn Val Val Ile Tyr Gly Thr Asp Arg Trp
           115                 120                 125
   Ala Thr Gly Thr Arg Thr Gly Ala Val Gly Ile Pro Ala Ser Pro Pro
       130                 135                 140
   Ser Glu Lys Tyr Pro Thr Ala Gly Lys Ile Lys Leu Val Thr Ala Lys
   145                 150                 155                 160

## Description

### TECHNICAL FIELD

This invention is in the field of assays for influenza virus hemagglutinin *e.g.* for analysing vaccines.

### BACKGROUND ART

The standard assay for hemagglutinin (HA) content in inactivated influenza vaccines is based on single radial immunodiffusion ("SRID") [1,2], which was recommended by the WHO in 1978 to replace tests based on agglutination of erythrocytes.

Although the SRID assay is well established, it is slow to perform, has poor dynamic range, considerable variability, and it can take a long time to prepare and calibrate the required specific anti-HA serum. Moreover, it cannot be used for in-process monitoring. Thus people have looked for non-SRID assays for quantifying influenza HA.

For instance, reference 3 discloses an assay which uses ultrafiltration followed by RP-HPLC. Reference 4 discloses a method where HA is reduced in the presence of a detergent, alkylated to protect its sulfhydryl groups, applied to a RP-HPLC column, and then eluted with an ion pairing agent in an organic mobile phase. Reference 5 uses electrophoretic separation of deglycosylated samples in conjunction with densitometry. ELISA assays have also been reported *e.g*. reference 6.

Some alternatives to SRID involve modification of the samples (*e.g*. deglycosylation, denaturation, or sulfhydryl derivatisation). Some also lack strain discrimination, so they are not useful for analysis of products containing multiple different hemagglutinins (*e.g*. seasonal vaccines, which usually contain HA from 3 viral strains). Furthermore, approaches based on some physicochemical techniques can require highly-trained staff.

There remains a need for further and improved alternatives to the SRID assays for quantifying influenza virus HA.

### DISCLOSURE OF THE INVENTION

The invention quantifies HA using a method which comprises two key steps: HA is captured using a lectin, such as snowdrop lectin; and captured HA is quantified using an immunoassay. This assay can conveniently be performed in an ELISA format. The method is specific, is robust in the face of other components in the sample, and is more rapid and more sensitive than the current SRID assay. Conveniently, however, it can utilise the reagents (*e.g.* anti-HA antibodies) which are currently prepared by public health authorities for use in the SRID assay, and can be used to quantify different HA proteins in a single captured sample by using different anti-HA antibodies for the immunoassay.

Thus the invention provides a method for analysing a vaccine comprising steps of (a) detecting influenza virus hemagglutinin in the vaccine, or a sample thereof, comprising steps of: (i) contacting the sample with a lectin which is immobilised on a solid support, to provide captured hemagglutinin; and (ii) detecting the captured hemagglutinin by immunoassay; and (b) using the results of step (a) to calculate the hemagglutinin concentration in the vaccine. The detection step is ideally a quantitative step, thereby permitting the amount of HA in the original sample to be quantified.

The invention also provides a method for providing a bulk vaccine with a desired hemagglutinin concentration, comprising steps of: (a) analysing hemagglutinin in the vaccine, or in a sample thereof, by a method of the invention; (b) using the results of step (a) to calculate the hemagglutinin concentration in the bulk vaccine; and (c) using the results of step (b) to dilute the bulk vaccine to give the desired hemagglutinin concentration.

The invention also provides a method for providing a vaccine for patient use, comprising steps of: preparing a bulk vaccine with a desired hemagglutinin concentration by a method of the invention; and then extracting one or more unit doses of vaccine from the diluted bulk.

The invention also provides a method for providing a bulk adjuvanted vaccine comprising steps of: preparing a bulk vaccine with a desired hemagglutinin content by a method of the invention; and then mixing the diluted bulk vaccine with an adjuvant.

The invention also provides a method for providing an adjuvanted vaccine for patient use, comprising steps of: providing a bulk adjuvanted vaccine by a method of the invention; and then extracting one or more unit doses of vaccine from the bulk.

The lectin-based capture step is useful for purification of HA even without any detection or quantification. Thus, the invention also provides a method for purifying influenza virus hemagglutinin in a sample, comprising a step of contacting the sample with a lectin which is immobilised on a solid support, wherein the lectin is a mannose-binding lectin, with the proviso that the lectin is not a conA lectin. HA captured by the lectin can then be eluted to provide purified HA material. Thus the lectin-based capture step can be used as a preparative technique for influenza HA *e.g.* to prepare HA standards with high purity. Such standards can be useful in the preparation of influenza vaccines.

The invention may use an affinity chromatography support or column to which the lectin (*e.g.* GNA lectin) is immobilised, for use in affinity purification of influenza virus HA. Such materials are already available (*e.g*. agarose-linked GNA lectin from Vector Laboratories) but their use in purification of influenza virus HA is new.

Thus, the methods of the invention prepare purified HA that preferably has a known HA concentration such that it can be used as a standard reagent *e.g.* as part of vaccine manufacture. This purified HA is particularly useful when prepared from influenza viruses grown in cell culture *e.g.* in MDCK cells. For instance, the purified HA can be used as a control or as a calibration reagent for other assays *e.g.* for SRID. Usefully, the purified HA is substantially free from other proteins *(e.g.* from influenza virus neuraminidase) such that HA is substantially the only protein component in the material.

### Lectin capture of HA

Methods of the invention use an immobilised lectin to capture influenza virus HA. The lectin ideally binds to mannose residues, such as α-1,3-linked mannose residues. Various mannose-binding lectins are known in the art, and these are typically plant lectins (and, in particular, monocot lectins) such as those from snowdrop (*Galanthus nivalis*, 'GNA' lectin), from daffodil (*Narcissus pseudonarcissis*, 'NPA' lectin), from garlic (*Allium sativum*, 'ASA' lectin), and from lentil (*Lens culinaris*, 'LCH' lectin). Concanavalin A (from *Canavella ensiformis*, 'ConA' lectin) is a commonly-used mannose-binding lectin.

The lectin for use with the invention for quantifying HA is preferably a mannose-binding lectin, but not a ConA lectin. The lectin is preferably not a galactose-binding lectin. Examples of galactose-binding lectins include the β-galactose specific *Erythrina christagalli* lectin (ECL) and the α-galactose specific *Euonymus europaeus* lectin (EEL).

The lectin for use with the invention for purifying HA is a mannose-binding lectin, but not a conA lectin.

Mannose is a common carbohydrate residue in glycoproteins, *e.g*. HA and NA. It was unclear whether mannose-binding lectins would be able to specifically and efficiently capture HA from other viral components. Surprisingly, the inventors found that mannose-binding lectin (*e.g.* GNA lectin) is able to capture HA with high specificity. In particular, the mannose-binding lectin is able to capture HA of different influenza virus strains, independent of their HA glycosylation levels. The inventors also found that mannose-binding lectin is also able to capture HA of influenza viruses that had been grown in various hosts, *e.g.* eggs and MDCK cells. Hence, variation of host cell dependent glycosylation of viral envelope glycoproteins does not appear to affect the capture specificity of mannose-binding lectin to HA.

The lectin for use with the invention ideally has a higher binding affinity to HA than to other viral envelope proteins *(i.e.* not HA), empty capsids, nucleic acids and/or proteins from hosts *(i.e.* for growing the influenza virus). The preferred lectin for use with the invention is GNA, originally prepared from snowdrop bulbs. This lectin naturally is a homotetramer and at least six different amino acid sequence variants or isoforms have been reported for GNA lectin (SEQ ID NOs: 1 to 6 herein). Its 3D structure has been determined. Each monomer in the tetramer has three sub-domains, each of which can bind to a mannose to give a 12-valent lectin. Unlike many mannose-binding lectins (a) it is not a metalloprotein and its binding activity does not require bivalent cations (b) it does not also bind to α-linked glucose residues. As shown below, GNA lectin efficiently captures influenza virus HA to provide purified material *e.g.* for detection, for quantification, or for use as a standard antigen, *etc.*

The invention can use a wild-type GNA lectin or can use modified forms which still retain the useful HA-binding activity of wild-type GNA lectin. Thus the lectin used with the invention can comprise any of amino acid sequences SEQ ID NOs: 1 to 6, which are variants of the GNA lectin precursor. As an alternative, the lectin can (i) comprise a fragment of at least *n* consecutive amino acids from any of SEQ ID NOs: 1 to 6; and/or (ii) comprise an amino acid sequence with at least *x*% sequence identity to any of amino acid sequences SEQ ID NOs: 1 to 6, provided that it can bind to influenza virus HA, where *x* is 70 or more *e.g.* 75, 80, 85, 88, 90, 92, 94, 95, 96, 97, 98, 99 or more (*e.g.* 100) and n is 7 or more (*e.g*. 8, 10, 12, 14, 16, 18, 20, 25, 30, 35, 40, 50, 60, 70, 80, 90 or more). Suitable fragments include those lacking the native signal peptide of SEQ ID NOs: 1 to 6 (*e*.*g*. lacking amino acids 1-26 of SEQ ID NO: 6). Various GNA-related lectins are known in the art [8]. However, none of the known GNA, variants or isoforms thereof, and GNA-related lectins has been used as part of influenza vaccine manufacture, *e.g*. for purifying, detecting or quantifying HA.

The lectin will typically be used in the form of an oligomer *e.g*. a tetramer.

For use with the invention the lectin is immobilised on a solid support. Appropriate supports can be chosen depending on the intended format of any assay *e.g*. the support might be a solid plate, a bead, a column support, *etc.* A preferred support is a plate suitable for use in an ELISA assay. For instance, the lectin can be immobilised on a plastic multiwell plate *e.g.* a 96-well or 384-well microtitre plate. A polystyrene support is typical for ELISA, although polylactate supports can also be used.

Other solid supports, *e.g*. those typically used in affinity chromatographic methods, include packed beds, membrane adsorbers and monoliths [9]. For packed beds, the matrices typically contain resins (beads) and are usually made of polymer or inorganic resin materials. Polymer-based beads can be made of natural polymers (*e.g*. agarose, agarose-dextran composites, cellulose and dextran) or synthetic polymers (*e.g*. polyacrylamide, polystyrene divinylbenzene, derivatives of polyacrylamide and polymethacrylate). Examples of inorganic resin materials include hydroxyapatite, silica and controlled-pore glass. Beads can be spherical and non-spherical shaped and are available as porous, nonporous and solid-core resins. Pore sizes of conventional porous beads range from 10 to 100 nm. Monoliths include those made of polymethacrylate copolymers, polyacrylamide, polystyrene, polystyrene-divinylbenzene, modified cellulose and silica. Monoliths can be disk-, tube- and rod-shaped with various pore sizes. Suitable membrane adsorbers include cellulose, polyamine, polysulfone, hydrazide and composite membranes (*e.g*. polyethylene oxide and polyethersulfone coated with hydroxyethyl-cellulose. The membrane adsorbers can be flat sheets, hollow fibres or radial-flow devices.

Immobilisation of the lectin can be achieved in various ways. For many assay formats (*e.g*. ELISA) it can be sufficient to adsorb the protein non-specifically to a support; for other formats the lectin can be immobilised by means of a covalent linker using standard chemistry *e.g*. as commonly used for preparing affinity chromatography supports.

For embodiments where captured HA will be assayed it is not necessary to elute the HA before it is assayed (but for some non-ELISA assay formats such elution might nevertheless be desired or necessary). For embodiments where HA is being purified then the HA must be eluted after capture, and this can be achieved by contacting the immobilised lectin with a high affinity ligand for the lectin *e.g.* with free mannose or mannose-containing oligosaccharide, as typical in affinity chromatography.

### Immunoassay of HA

Most embodiments of the invention include a step in which captured HA is detected using an immunoassay. The immunoassay is ideally a quantitative immunoassay, such that the detection step permits the amount of HA in the sample to be determined.

The immunoassay will use an anti-HA antibody, which can be polyclonal or monoclonal. Ideally the antibody is strain-specific, such that a particular HA can be detected even in the presence of other HA species. For instance, in a current seasonal vaccine (A/H1N1, A/H3N2, and B strains) the invention (like SRID) can permit the measurement of each of the three HA proteins even in the presence of each other by using three different anti-HA antibodies, each of which is specific for only one of the HA proteins. Thus the invention can involve universal capture (via a lectin which binds to each HA) but specific detection (via an immunoassay which can distinguish between different HA proteins which are present in the sample). In some embodiments, however, particularly those for analysing monovalent HA materials (*e.g*. monovalent bulk vaccines) the invention can readily be performed using an anti-HA antibody (*e.g.* a monoclonal antibody) that is broadly reactive with HA from multiple HA types or with HA from multiple strains with the same HA type.

Suitable anti-HA antibodies are widely available. For example, public health agencies prepare antisera for use in vaccine analysis in each influenza season. These antisera can be used with the invention, but other anti-HA antibodies can also be used. The antibody may be modified to permit attachment of an enzyme for ELISA purposes (see below).

The anti-HA antibody is ideally an IgG antibody. Sheep IgG is particularly useful with the invention. Suitable sheep IgG can be purified from reference sera provided as SRID reagents by public health authorities.

The immunoassay is ideally performed *in situ*, that is with HA which is still bound to lectin. In another embodiment, the HA is eluted from the lectin and the immunoassay is performed post-elution, *i.e.* HA is no longer bound to lectin.

### ELISA format

A preferred embodiment of the invention is an ELISA format, and more preferably a sandwich ELISA format. This format is useful because reagents, equipment and technicians are very familiar with it, and standard protocols (*e.g.* immobilisation, capture, washing, detection, calibration, *etc*.) can easily be used to provide reliable quantitative results.

In the ELISA format the immobilised lectin captures HA, and the captured HA is then exposed to anti-HA antibodies. The assay format can involve a primary antibody (which binds to the HA) and a secondary antibody (which binds to the primary antibody), with the secondary antibody comprising an enzyme. This primary/secondary arrangement means that the same enzyme-linked secondary antibody can be used multiple times, without needing to prepare a new enzyme-conjugated detection antibody for each new HA. In a preferred format, however, the ELISA uses a tagged anti-HA antibody, which is then contacted with an enzyme which binds to the tag. For example, a biotin-conjugated anti-HA antibody can bind to captured HA and then a streptavidin-conjugated enzyme is added. The enzyme which is now immobilised on the solid support is then used to catalyse a reaction which can easily be assayed, as in the standard ELISA technique.

The invention can use any enzyme which is suitable for use in ELISA *e.g.* any enzyme which can convert a substrate into a detectable product, such as a coloured, luminescent (*e.g*. fluorescent) or electrochemically-active product. Such enzymes include, but are not limited to, alkaline phosphatase (which can convert pNPP to a coloured soluble product) or a peroxidase such as horseradish peroxidase (which can convert tetramethyl benzidine to produce a coloured soluble product; other typical substrates include ABTS, OPD).

The invention utilises standard and known steps from existing ELISA protocols *e.g*. calibration, washing, negative controls, positive controls, replication, *etc.* The ELISA technique is ideally performed in a 96- or 384-well plate.

In some embodiments the invention can utilise the alternative ELISA protocol disclosed in reference 10. In this protocol the anti-HA antibody and the HA are mixed before contacting the HA with the lectin. In other embodiments, however, the standard ELISA protocol is used, in which the HA is captured by the lectin and then the anti-HA antibody is added. If the alternative protocol is used then the method should uses an anti-HA antibody which does not block the lectin's binding site on the HA, and it is straightforward to screen for monoclonal antibodies which meet this requirement.

The inventors found that ELISA can be sensitive to anti-host (*e*.*g*. eggs) antibodies, *e.g.* which can be found in NIBSC and CBER reference sera, and this could lead to inaccurate calibration and/or inaccurate quantification of a sample. The inventors found that this inaccuracy can be minimised if the virus to which anti-HA antibodies are raised and the HA (in the calibration standards or samples) are derived from different sources. For example, anti-HA raised against influenza viruses that had been grown in eggs (*i.e.* egg-derived influenza viruses) can be used to detect HA from influenza viruses that had been grown in cell culture (*i*.*e*. cell culture-derived HA). Conversely, anti-HA antibodies raised against influenza viruses that had been grown in cell culture (*i*.*e*. cell culture-derived influenza viruses) can be used to detect HA from influenza viruses that had been grown in eggs (*i.e*. egg-derived HA).

Thus, the invention provides a method for quantifying the HA in a sample comprising cell culture-derived HA using the ELISA format of the invention, wherein the ELISA is calibrated using cell culture-derived HA and anti-HA antibodies raised against egg-derived influenza viruses. Useful anti-HA antisera include those provided by NIBSC or CBER.

Conversely, the invention provides a method for quantifying the HA in a sample comprising egg-derived HA using the ELISA format of the invention, wherein the ELISA is calibrated using egg-derived HA and anti-HA antibodies raised against cell culture-derived influenza viruses.

The antigen calibration standard for the ELISA typically has a known HA concentration, *e.g.* as determined by ELISA, SRID or other conventional assays such as absorption at 280nm and BCA assay.

### Purification of HA

The methods of the invention use a mannose-binding lectin that is not a conA lectin in a capture step to purify HA from a sample. Thus, the invention provides a method for purifying influenza virus HA in a sample, comprising a step of contacting a sample with a lectin which is immobilised on a solid support. For example, the invention uses lectin as the affinity ligand in affinity chromatographic methods. In one embodiment, the invention uses lectin immobilised on an affinity chromatography support or column for recovery and purification of HA from a sample.

Ideally, the purified HA protein retains its immunogenicity (*i.e.* it can elicit antibody production when administered in a subject) and activity (*e.g.* it can agglutinate erythrocytes). The purified HA can be used as a vaccine component.

Alternatively, the purified HA can be used as a control or as a calibration standard, *e.g*. in immunoassays (*e.g*. ELISA or SRID), for the preparation of influenza vaccines.

Preferably, the methods of the invention use a GNA lectin, a variant or isoform thereof, or a GNA-related lectin, as discussed above.

The methods of the invention can use a sample comprising antigens from influenza viruses that had been grown in eggs or mammalian cells. Preferably, the mammalian are not MDCK or Vero cells. The methods of the invention can use a sample comprising antigens from at least one influenza virus strain. Preferably, the sample comprises more than one influenza virus strains.

The methods of the invention can comprise treating a sample comprising influenza virus prior to purifying HA, *e.g.* inactivating and/or splitting the influenza virus. Thus, a method for purifying HA can use a sample comprising inactivated viruses, including whole virons, split virions, purified surface antigens or virosomes. Preferably, the sample does not contain whole virions. Preferably, the sample does not contain whole virions of strains A/Puerto Rico/8/34, A/Wisconsin/67/2005 and B/Malaysia/2506/2004.

Alternatively, the methods of the invention can use a sample comprising live influenza viruses. The invention also provides an influenza virus vaccine comprising HA, wherein the vaccine is (i) a vaccine prepared from a HA bulk, wherein the HA in the bulk has been quantified in a method that uses a HA calibration standard prepared according to the methods of the invention, and/or (ii) part of a batch of vaccines in which at least one vaccine from the batch has been quantified in a method that uses a HA calibration standard prepared according to the methods of the invention.

The purified HA sample is ideally substantially free from other viral components, such as free from neuraminidase (NA), free viral envelope proteins, empty capsids and nucleic acids. The purified HA is also substantially free from host (*e.g*. egg or cell) proteins, nucleic acids and reagents from cell culture (if viruses were grown in cell culture). Ideally, the purified sample has minimal host (*e.g.* egg or cell such as MDCK cell) proteins.

Ideally, the invention uses mannose-binding lectin but not a conA lectin as the affinity ligand in affinity chromatographic methods that are known in the art for purifying proteins, *e.g*. spin columns or FPLC [*e.g.* References 11,12]. Chromatography purification steps typically include incubating a sample with the affinity ligand to capture the analyte, and eluting the captured analyte. Optional wash steps can be included.

For example, the purification methods of the invention may include equilibrating a solid support on which lectin is immobilised (*e.g*. an affinity chromatography support or column), adsorbing lectin on the support, incubating a sample with the immobilised lectin to capture HA, and eluting the captured HA as purified HA proteins. Optionally, the sample can be mixed with octylglucoside (*i.e.* n-Octyl-beta-D-glucopyranoside) prior to incubating with the immobilised lectin. For example, octylglucoside is present at a final concentration of 1.5% (w/v).

Solid supports useful for the invention are discussed above, and are also discussed in reference 13. Suitable supports include polymer matrices, *e.g*. those having a mean pore diameter of about 100 nm and ligand density between 4.0 and 5.0 mg/ml. Lectin immobilized on polymer matrices can be purchased from GALAB Technologies GmbH. Supports containing agarose, *e.g*. Crosslinked Sepharose 4B, is also suitable for the invention, particularly those with a ligand density of about 3 mg/ml of settled gel. Lectin immobilised on crosslinked sepharose are commercially available, *e.g*. from Vector Laboratories Inc. Actigel ALD (Sterogene Bioseparation Inc.) with a bead size of about 60-160 µm and about 4% agarose may also be suitable.

Suitable solid supports containing activated porous glass particles may also be useful, *e.g*. Trisopor® 4000 Diol from VitraBio GmbH, which has a particle size from about 125 to 200 µm and a pore size of about 350-400 nm. Other useful solid supports include those containing cellulose beads, *e.g.* Cellufine™ Formyl (Chisso Corporation) having a particle size of about 125-210 µm. Reinforced cellulose membranes are also suitable, *e.g*. Sartobind® Epoxy membrane adsorbers (Sartorius AG) having pore size *e.g.* about 0.45 µm.

Immobilisation of the mannose-binding lectin can be achieved in various ways. For example, the mannose-binding lectin can be immobilised by means of a covalent linker using standard chemistry *e.g.* as commonly used for preparing affinity chromatography supports. Typically, the immobilised lectin density is about 3-8 mg/ml adsorbent. The skilled person would be able to determine the appropriate immobilised lectin density according to known techniques in the art.

Elution of the captured HA may be achieved by contacting the immobilised lectin with a high affinity ligand for the lectin *e.g.* with free mannose or mannose-containing oligosaccharide, as typical in affinity chromatography. The captured HA can be eluted using α-methyl-mannopyranoside.

Preferably, at least 75%, 80%, 90% or 95% of the HA (mass) is recovered from the total HA (mass) in the starting material using purification methods of the invention.

The HA in the purified sampled can be quantified, *e.g*. as determined by ELISA of the invention or other assays, *e.g*. ELISAs that use antibodies to capture HA, SRID, absorption at 280nm and BCA assay.

The purified HA may be treated by one or more additional steps, *e.g.* size exclusion chromatography, ion-exchange chromatography, HPLC, chromatography using heparinized or sulphated matrices, *etc.* The methods of the invention may include further tests commonly used in the art to assess immunogenicity, activity or toxicity of the purified HA.

When the purified HA is used as a vaccine, the vaccine ideally does not contain lectin. In embodiments where the purified HA sample contains lectin, additional steps can be included to remove the lectin. Vaccines containing <10ng (*e.g.* <lng, <100pg) host cell DNA per 15µg of hemagglutinin are preferred, as are vaccines containing <10ng (*e.g.* <lng, <100pg) host cell DNA per 0.25ml volume. Vaccines containing <10ng (*e.g.* <lng, <100pg) host cell DNA per 50µg of hemagglutinin are more preferred, as are vaccines containing <10ng (*e.g.* <lng, <100pg) host cell DNA per 0.5ml volume.

### The sample

The sample which is analysed or purified using the invention contains (or is at least suspected to contain) influenza virus hemagglutinin (HA). SRID is often performed on material containing HA from several strains (*e.g*. trivalent material). The invention can be used with multivalent samples (*e.g*. 2-valent, 3-valent, 4-valent or more) and can separately detect and/or quantify the antigen from each strain by using appropriate detection reagents, which are already known for use with SRID. In other embodiments, however, the invention can be used to analyse or purify HA in monovalent samples.

The invention can be used with various types of sample. It can be used to analyse a final vaccine, but as the analytical method is destructive this will usually be a single vaccine from a batch, with analytical results indicating properties of the batch. The sample may have been taken from bulk vaccine material, with analytical results indicating properties of the bulk as a whole. In other embodiments the sample may even be virus-containing fluids *e.g*. it may be virus-containing harvest fluids from a cell culture or from eggs. The sample may thus be starting material, final vaccine material, or any manufacturing intermediate between virus culture and final vaccine. Preferably, the sample has minimal proteins from the host (*i.e.* the host for growing the influenza virus).

The sample will typically include HA from influenza virions but, as an alternative, it may include HA which was expressed in a recombinant host (*e.g.* in an insect cell line using a baculovirus vector) and purified [14,15,16] or may be in the form of virus-like particles (VLPs; *e.g.* see references 17 and 18). In general, however, antigens will be from virions and so the sample may, in addition to HA, include other influenza virus proteins (*e.g*. PB1, PB2, PA, NP, NA, M1, M2, NS1 and/or NS2 proteins), and may also include influenza virus lipids.

Virion-derived influenza virus antigens are based either on live virus or on inactivated virus (*e.g*. see chapters 17 & 18 of reference 19). Although the invention can be used to determine HA levels from live virus, dosing of live vaccines is based on median tissue culture infectious dose rather than HA content, and so the invention will usually be used to determine HA levels in an inactivated vaccine.

Inactivated vaccines may be based on whole virions, 'split' virions, purified surface antigens (including HA and, usually, also including neuraminidase) or virosomes (nucleic acid free viral-like liposomal particles [20]). The invention can be used with all such vaccines. The BEGRIVAC™, FLUARIX™, PREPANDRIX™, FLUZONE™ and FLUSHIELD™ products are split vaccines. The FLUVIRIN™, AGRIPPAL™, FLUAD™ and INFLUVAC™ products are surface antigen vaccines.

The INFLEXAL V™ and INVAVAC™ products are virosome vaccines. The invention is most useful for measuring HA content in split and surface antigen vaccines. Thus, where the text refers to capture of HA, this can be capture of free HA, or can be capture of HA in combination with other components of influenza virus *e.g*. HA as part of an influenza virion. Similarly, where the text refers to detection of HA, this can be detection of free HA, or can be detection of HA in combination with other components of influenza virus *e.g*. detection of HA as part of an influenza virion. In preparative purification methods of the invention, however, the HA is ideally prepared as purified HA protein *e.g*. which is substantially free from other viral components, such as free from neuraminidase.

The invention can be used with HA from any influenza virus type, including both influenza A virus and influenza B virus (irrespective of origin, *e.g*. human, equine, avian, canine, swine flu). The invention is therefore suitable for analysing or purifying HA from a variety of influenza strains. The invention is preferably used for human influenza viruses, particularly strains for inclusion in human vaccines.

For influenza A viruses the invention can be used with HA from any known HA subtype (H1, H2, H3, H4, H5, H6, H7, H8, H9, H10, H11, H12, H13, H14, H15 or H16), and it is particularly useful with H1, H3 and H5 strains. The strain may have any of NA subtypes N1, N2, N3, N4, N5, N6, N7, N8 or N9. For example, the invention can be used to analyse material from a H1N1 strain, a H1N2 strain, a H3N2 strain, a H5N1 strain, *etc.* The invention is very useful for analysing trivalent vaccines *e.g*. containing HA from each of a A/H1, A/H3 and B virus.

For influenza B viruses the invention can be used with HA from a B/Victoria/2/87-like strain or a B/Yamagata/16/88-like strain. These two groups of strains are usually distinguished antigenically, but differences in amino acid sequences have also been described for distinguishing the two lineages *e.g*. B/Yamagata/16/88-like strains often (but not always) have HA proteins with deletions at amino acid residue 164, numbered relative to the 'Lee40' HA sequence [21].

HA which is analysed by methods of the invention may be full-length precursor HA, known as HA0. In some embodiments, however, the HA is a fragment of HA0. HA0 can be fragmented by serine proteases, such as trypsin, to yield a N-terminal fragment (HA1) and a C-terminal fragment (HA2), which can remain joined by a disulfide bridge. Thus the methods of the invention may purify HA0, HA1 or HA2, and may involve detection and analysis of only one such fragment (*e.g*. of HA1). Other HA fragments which might be purified and analysed include, for instance, the fragment released by bromelain treatment, or fragments obtained by treatment with both bromelain and trypsin. Typically the methods will purify HA1, and thus at least the RP-HPLC step should be performed under reducing conditions (*e.g*. using DTT) to ensure HA1 and HA2 are separated, and optionally may involve a pre-purification digestion step (*e.g*. using trypsin) to ensure full cleavage of HA1 from HA2 (but this digestion is often unnecessary, and the presence of any full-length HA0 can easily be tested to determine if digestion would be useful).

HA in the sample may include a hyper-basic region around the HA1/HA2 cleavage site, but in other embodiments this region is absent.

HA in the sample may have a binding preference for oligosaccharides with a Sia(α2,6)Gal terminal disaccharide compared to oligosaccharides with a Sia(α2,3)Gal terminal disaccharide, or *vice versa*, or it may show no such preference. Assays for this preference are discussed in reference 22. Human influenza viruses bind to receptor oligosaccharides having a Sia(α2,6)Gal terminal disaccharide (sialic acid linked α-2,6 to galactose), but eggs and Vero cells have receptor oligosaccharides with a Sia(α2,3)Gal terminal disaccharide.

In some embodiments the HA has a different glycosylation pattern from the patterns seen in egg-derived viruses. Thus the HA may include glycoforms that are not seen in chicken eggs. Useful HA includes canine glycoforms *e.g.* the virus may have been grown in MDCK cells. Other useful HA includes human glycoforms *e.g*. the virus may have been grown in PER.C6 cells. Other useful HA includes simian glycoforms *e.g*. the virus may have been grown in Vero cells. These cell lines are widely available *e.g*. from the American Type Cell Culture (ATCC) collection, from the Coriell Cell Repositories, or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. One suitable MDCK cell line is 'MDCK 33016', deposited as DSM ACC 2219 [23].

The most preferred cell lines for growing influenza viruses are MDCK cell lines [23-26], derived from Madin Darby canine kidney. The original MDCK cell line is available from the ATCC as CCL-34, but derivatives of this cell line may also be used. For instance, reference 23 discloses a MDCK cell line that was adapted for growth in suspension culture ('MDCK 33016', deposited as DSM ACC 2219). Similarly, reference 27 discloses a MDCK-derived cell line that grows in suspension in serum-free culture ('B-702', deposited as FERM BP-7449). Reference 28 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 29 discloses MDCK cell lines with high susceptibility to infection, including 'MDCK.5F1' cells (ATCC CRL-12042). Any of these MDCK cell lines can be used.

The inventors found that mannose-binding lectin is able to specifically capture HA of influenza viruses that had been grown in various hosts, e.g. eggs and MDCK cells. Thus, the methods of the invention are able to capture HA independent of their HA glycosylation profiles. HA in the sample may be from a wild-type virus or from a reassortant virus, particularly for influenza A virus. A reassortant virus may have been obtained by reverse genetics techniques. Thus a sample may include HA from one virus strain but other influenza antigens (*e.g.* PB1, PB2, PA, NP, M1, M2, NS1 and/or NS2 proteins) from a different strain *e.g*. from A/PR/8/34, from A/AA/6/60, or from A/WSN/33.

The HA concentration in a sample will usually be between 0.1µg/ml and 10mg/ml *e.g*. between 1µg/ml and 1mg/ml, or between 10µg/ml and 100µg/ml. In some embodiments the concentration is about 30µg/ml, about 15µg/ml or about 7.5µg/ml. The invention can be used to quantify HA in this range.

The sample may include serum components but is preferably free from such components.

The sample may include egg proteins (*e.g*. ovalbumin and ovomucoid) and/or chicken DNA, but in some embodiments is free from these components *e.g*. when virus was grown in cell culture.

The sample may include DNA *e.g.* chicken DNA or mammalian DNA (*e.g.* derived from MDCK cells, Vero cells, PER.C6 cells, *etc.*). Ideally, however, a sample contains less than 600ng of DNA per mg of HA (preferably less than 60ng, and more preferably less than 6ng).

The sample preferably includes no viral proteins except for influenza virus proteins.

The sample may include detergent *e.g*. a polyoxyethylene sorbitan ester surfactant (known as 'Tweens' *e.g*. polysorbate 80), an octoxynol (such as octoxynol-9 (Triton X-100) or -10, or t-octylphenoxypolyethoxyethanol), a cetyl trimethyl ammonium bromide ('CTAB'), or sodium deoxycholate, particularly for a split or surface antigen vaccine sample. The detergent may be present only at trace amounts *e.g*. residual from antigen manufacture. Other sample components in trace amounts could be antibiotics (*e.g*. neomycin, kanamycin, polymyxin B).

The invention is described herein in relation to influenza virus HA, but it will be apparent that the principles of the invention can equally be applied to further viruses and antigens.

### Downstream steps

Methods of the invention permit the measurement of HA concentration in material of interest. This material, particularly a bulk vaccine, can then be diluted to give a desired final HA concentration. Thus a method of the invention may include a further step of diluting a vaccine based on the results of HA quantification, and the invention provides a method for analysing a vaccine comprising steps of: (a) quantifying HA in the vaccine, or in a sample thereof, by a method as disclosed herein; and (b) using the results of step (a) to calculate the HA concentration in the vaccine. The invention also provides a method for providing a bulk vaccine with a desired HA concentration, comprising said steps (a) and (b), and a further step (c) using the results of step (b) to dilute the bulk vaccine to give the desired HA concentration. Unit doses of the diluted bulk vaccine can then be extracted, and so the invention provides a method for providing a vaccine for patient use, comprising said steps (a) to (c), and a further step (d) extracting one or more unit doses of vaccine from the diluted bulk, each unit dose having a desired HA content. The extracted material can be placed into a container *e.g*. a vial or syringe.

Diluted bulk can be mixed with other components, such as an adjuvant, prior to unit dose extraction. Thus the invention provides a method for providing a bulk adjuvanted vaccine comprising said steps (a) to (c), and a further step (d) mixing the diluted bulk vaccine with an adjuvant. The invention also provides a method for providing an adjuvanted vaccine for patient use, comprising said steps (a) to (d), and a further step (e) extracting one or more unit doses of vaccine from the diluted bulk, each unit dose having a desired HA content. The extracted material can be placed into a container e.g. a vial or syringe.

As an alternative to mixing an extracted dose with an adjuvant, the extracted dose may instead be packaged as a first kit component in combination with a second kit component, wherein the second kit component is a vaccine adjuvant. The two kit components can be combined at the time of use to give an adjuvanted vaccine. The kit allows the adjuvant and the antigen to be kept separately until the time of use (*e.g.* as in the PREPANDRIX™ product). The components are physically separate from each other within the kit, and this separation can be achieved in various ways. For instance, the two components may be in two separate containers, such as vials. The contents of the two vials can then be mixed *e.g*. by removing the contents of one vial and adding them to the other vial, or by separately removing the contents of both vials and mixing them in a third container. In one arrangement, one of the kit components is in a syringe and the other is in a container such as a vial. The syringe can be used (*e.g*. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration. In another arrangement, the two kit components are held together but separately in the same syringe *e.g.* a dual-chamber syringe [30]. When the syringe is actuated (*e.g*. during administration to a patient) the contents of the two chambers are mixed. This arrangement avoids the need for a separate mixing step at the time of use.

Whether antigen and adjuvant are mixed during manufacture or at the time of delivery to a patient, antigen will generally be aqueous and so the mixing step involves mixing two liquids. The volume ratio of the two liquids for mixing can vary (*e.g*. between 5:1 and 1:5) but is generally about 1:1. Thus two kit components may include substantially the same volume of liquid as each other.

The adjuvant can enhance the immune responses (humoral and/or cellular) elicited in a patient who receives the composition. A typical adjuvant for this purpose is an oil-in-water emulsion. Various suitable emulsions are known, and they typically include at least one oil and at least one surfactant, with the oil(s) and surfactant(s) being biodegradable (metabolisable) and biocompatible. The oil droplets in the emulsion are generally less than 5µm in diameter, and advantageously the emulsion comprises oil droplets with a sub-micron diameter, with these small sizes being achieved with a microfluidiser to provide stable emulsions. Droplets with a size less than 220nm are preferred as they can be subjected to filter sterilization.

The emulsion can include oils from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g*. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolisable and may therefore be used in the practice of this invention. The procedures for separation, purification, saponification and other means necessary for obtaining pure oils from animal sources are well known in the art. Most fish contain metabolisable oils which may be readily recovered. For example, cod liver oil, shark liver oils, and whale oil such as spermaceti exemplify several of the fish oils which may be used herein. A number of branched chain oils are synthesised biochemically in 5-carbon isoprene units and are generally referred to as terpenoids. Shark liver oil contains a branched, unsaturated terpenoid known as squalene, 2,6,10,15,19,23-hexamethyl-2,6,10,14,18,22-tetracosahexaene. Other preferred oils are the tocopherols, including DL-α-tocopherol. Emulsions comprising squalene are particularly preferred. Mixtures of oils can be used.

Surfactants can be classified by their 'HLB' (hydrophile/lipophile balance). Preferred surfactants of the invention have a HLB of at least 10, preferably at least 15, and more preferably at least 16. Examples of suitable surfactants include, but are not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate; polysorbate 80), Span 85 (sorbitan trioleate), lecithin and Triton X-100. Inclusion of polysorbate 80 is preferred.

Mixtures of surfactants can be used *e.g*. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [31-33], as described in more detail in Chapter 10 of ref. 34 and chapter 12 of ref. 35. The MF59 emulsion advantageously includes citrate ions *e.g*. 10mM sodium citrate buffer.
- An emulsion comprising squalene, a tocopherol, and polysorbate 80. The emulsion may include phosphate buffered saline. These emulsions may have from 2 to 10% squalene, from 2 to 10% tocopherol and from 0.3 to 3% polysorbate 80, and the weight ratio of squalene:tocopherol is preferably ≤1 (*e.g.* 0.90) as this can provide a more stable emulsion. Squalene and polysorbate 80 may be present volume ratio of about 5:2 or at a weight ratio of about 11:5. Thus the three components (squalene, tocopherol, polysorbate 80) may be present at a weight ratio of 1068:1186:485 or around 55:61:25. One such emulsion ('AS03') can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g*. with an average diameter of between 100 and 250nm, preferably about 180nm. The emulsion may also include a 3-de-O-acylated monophosphoryl lipid A (3d-MPL). Another useful emulsion of this type may comprise, per human dose, 0.5-10 mg squalene, 0.5-11 mg tocopherol, and 0.1-4 mg polysorbate 80 [36] *e.g.* in the ratios discussed above.
- An emulsion of squalene, a tocopherol, and a Triton detergent (*e.g*. Triton X-100). The emulsion may also include a 3d-MPL (see below). The emulsion may contain a phosphate buffer.
- An emulsion comprising a polysorbate (*e.g.* polysorbate 80), a Triton detergent (*e.g.* Triton X-100) and a tocopherol (*e.g*. an α-tocopherol succinate). The emulsion may include these three components at a mass ratio of about 75:11:10 (*e.g*. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [37] (0.05-1% Thr-MDP, 5% squalane, 2.5% Pluronic L121 and 0.2% polysorbate 80). It can also be used without the Thr-MDP, as in the "AF" adjuvant [38] (5% squalane, 1.25% Pluronic L121 and 0.2% polysorbate 80). Microfluidisation is preferred.
- An emulsion comprising squalene, an aqueous solvent, a polyoxyethylene alkyl ether hydrophilic nonionic surfactant (*e.g*. polyoxyethylene (12) cetostearyl ether) and a hydrophobic nonionic surfactant (*e.g*. a sorbitan ester or mannide ester, such as sorbitan monoleate or 'Span 80'). The emulsion is preferably thermoreversible and/or has at least 90% of the oil droplets (by volume) with a size less than 200 nm [39]. The emulsion may also include one or more of: alditol; a cryoprotective agent (*e.g.* a sugar, such as dodecylmaltoside and/or sucrose); and/or an alkylpolyglycoside. Such emulsions may be lyophilised.
- An emulsion having from 0.5-50% of an oil, 0.1-10% of a phospholipid, and 0.05-5% of a non-ionic surfactant. As described in reference 40, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 41, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion comprising a mineral oil, a non-ionic lipophilic ethoxylated fatty alcohol, and a non-ionic hydrophilic surfactant (*e.g*. an ethoxylated fatty alcohol and/or polyoxyethylene-polyoxypropylene block copolymer) [42].
- An emulsion comprising a mineral oil, a non-ionic hydrophilic ethoxylated fatty alcohol, and a non-ionic lipophilic surfactant (*e.g*. an ethoxylated fatty alcohol and/or polyoxyethylene-polyoxypropylene block copolymer) [42].
- An emulsion in which a saponin (*e*.*g*. QuilA or QS21) and a sterol (*e*.*g*. a cholesterol) are associated as helical micelles [43].

The importance of measuring HA content in vaccines, and of diluting bulk vaccines to a desired HA content, arises because inactivated influenza vaccines are standardised by their HA levels. Current vaccines typically contain about 30µg/ml of HA per strain, although lower doses are also used *e.g*. for children, or in emergency situations. Fractional doses such as ½ (*i.e.* 15µg/ml HA, as in FOCETRIA™), ¼ (*i*.*e*. 7.5 µg/ml, as in PREPANDRIX™ when administered) and ⅛ have been used [44,45], as have higher doses (*e.g.* 3x or 9x doses [46,47]).Thus dilution may be performed to give a HA concentration between 0.1 and 200µg/ml per influenza virus strain, preferably between 1 and 150µg/ml *e.g*. 1-90µg/ml, 1-20µgml, 0.1-15µg/ml, 0.1-10µg/ml, 0.1-7.5µg/ml, 0.5-5µg/ml, 3.75-15µg/ml *etc.* Particular post-dilution concentrations include *e.g.* about 90µg/ml, about 45µg/ml, about 30 µg/ml, about 15 µg/ml, about 10 µg/ml, about 7.5 µg/ml, about 5 µg/ml, about 3.8 µg/ml, about 3.75 µg/ml, about 1.9 µg/ml, about 1.5 µg/ml, *etc.* Lower concentrations (*e.g*. <30µg/ml) are most useful when an adjuvant is present in the vaccine. Although concentrations as high as 180µg/ml have been used in some studies (*e.g*. reference 48), compositions of the invention will usually have a HA concentration of ≤30µg/ml.

### General

The term "comprising" encompasses "including" as well as "consisting" *e.g*. a composition "comprising" X may consist exclusively of X or may include something additional *e.g.* X + Y.

The word "substantially" does not exclude "completely" *e*.*g*. a composition which is "substantially free" from Y may be completely free from Y. Where necessary, the word "substantially" may be omitted from the definition of the invention.

The term "about" in relation to a numerical value x is optional and means, for example, *x*±10%.

"GI" numbering is used above. A GI number, or "GenInfo Identifier", is a series of digits assigned consecutively to each sequence record processed by NCBI when sequences are added to its databases. The GI number bears no resemblance to the accession number of the sequence record. When a sequence is updated (*e.g*. for correction, or to add more annotation or information) then it receives a new GI number. Thus the sequence associated with a given GI number is never changed.

Unless specifically stated, a method comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a cell substrate is used for reassortment or reverse genetics procedures, it is preferably one that has been approved for use in human vaccine production *e.g.* as in Ph Eur general chapter 5.2.3.

Identity between polypeptide sequences is preferably determined by the Smith-Waterman homology search algorithm as implemented in the MPSRCH program (Oxford Molecular), using an affine gap search with parameters *gap open penalty=12* and *gap extension penalty=1*.

### BRIEF DESCRIPTION OF THE DRAWINGS

Figure 1 illustrates an ELISA of the invention, with GNA (triangles) immobilised on the surface of a support, binding to HA (star), to which biotinylated anti-HA IgG is then added.
Figure 2 shows a chromatogram of material purified by affinity chromatography using immobilised GNA with A/Brisbane/59/07 vaccine. The boxed fractions in the peak were pooled and analysed by SDS-PAGE, as shown in the marked lanes in Figure 3.
Figure 3 shows SDS-PAGE analysis of the fractions obtained from affinity chromatography using immobilised GNA with A/Brisbane/59/07 vaccine (as indicated in Figure 2). Left-hand 3 arrows: reduced and boiled material; right-hand 3 arrows: non-reduced non-boiled material.
Figure 4 shows OD₄₀₅ₙₘ using anti-B/Florida IgG (solid line) or anti-Solomon IgG (dotted line). The x-axis shows antigen dilution.
Figure 5 is like Figure 4, but shows analysis using IgG from sheep antisera produced by immunization with egg-produced HA of (5A) A/Brisbane/59/07 H1N1 or (5B) A/Uruguay/716/2007 H3N2. Samples were H1N1 NIBSC antigen standard (■), H3N2 NIBSC antigen standard (•), or H1N1 monovalent bulk from viruses grown in cell culture (▲).
Figure 6 is like Figure 5, except the analysis of different samples is shown. Analysis using IgG from sheep antisera produced by immunization with egg-produced HA of (6A) A/Brisbane/59/07 H1N1 or (6B) A/Uruguay/716/2007 H3N2 are shown. Samples were H1N1 CBER antigen standard (■), H3N2 CBER antigen standard (•), or H3N2 monovalent bulk from viruses grown in cell culture (▲).
Figure 7 is like Figures 4 and 5, but shows analysis for monovalent bulk vaccine prepared from viral strains having low HA glycosylation: (7A) a H5N1 strain; (7B) a H1N1 strain.

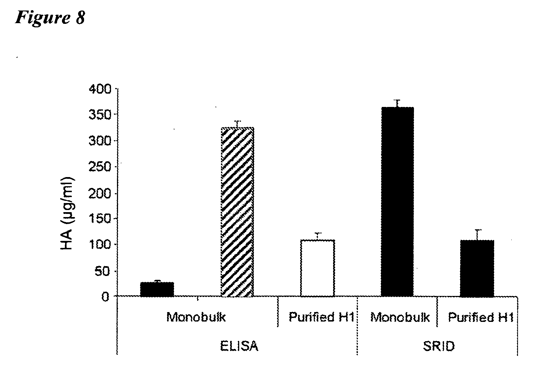

Figure 8 shows the HA concentration of samples obtained by ELISA and SRID using sheep antiserum against egg-produced A/Brisbane/59/07 (H1N1). The antigen samples were MDCK-produced A/Brisbane/59/07 monobulk and GNA-column purified HA from the monobulk. Black bars represent the concentration obtained when the egg-produced A/Brisbane/59/07 NIBSC standard reference was used as calibration standards; the striped bar indicates the concentration obtained when the purified MDCK-produced ABrisbane/59/07 H1 was used as calibration standards; and the white bars represent the concentration obtained when the A/Brisbane/59/07 (H1N1) monobulk was used as calibration standards.

### MODES FOR CARRYING OUT THE INVENTION

### ELISA format

An ELISA of the invention can easily be prepared. Standard protocols for preparing an ELISA assay can be used, but the capture reagent on the ELISA plate is a lectin such as GNA lectin. 100µl of influenza virus vaccine is applied to each well (in duplicate) and is incubated at 37°C for 30 minutes. Each well then receives 100µl of biotinylated anti-HA IgG, followed by a further 30 minutes of incubation. The plate is then washed 4 times, and 200µl streptavidin-conjugated alkaline phosphatase is added. The plate is incubated for 30 minutes at room temperature in the dark and is then again washed 4 times. 200µl of pNPP is then added and the enzymatic reaction is allowed to proceed for 30 minutes. Each well is then analysed to provide quantitative results. The overall process takes less than 3 hours, whereas a SRID assay takes at least 24 hours. Moreover, it can easily be performed by laboratory staff and does not require any treatment of the sample (*e.g*. no requirement for concentration, deglycosylation, denaturation, *etc.*).

The IgG used in the assay were purified from the sheep antisera provided by NIBSC or CBER using a G-protein column (HiTrap™ Protein G HP, GE Healthcare) following manufacturer instructions. IgG content in eluted fractions was determined by absorbance at 280 nm, considering that an absorbance of 1.4 OD indicates a 1 mg/ml IgG concentration. Purified fractions with OD>0.3 were pooled and dialyzed at +4°C overnight, with 4 liters of PBS 1x in a Slide-A-Lyzer Dialysis Cassettes, 10K MWCO (Pierce). During dialysis buffer was changed 3 times. The IgGs were concentrated at 1mg/ml by centrifugation at 3000 rpm using an AMICON ULTRA-15 Ultracel-10k column, then incubated 1 hour at room temperature with shaking with a 10x molar excess of NHS-PEG4-Biotin dissolved in PBS at a concentration of 3 mg/ml. After incubation IgGs were run down a PD-10 column equilibrated in PBS-2 mM EDTA (pH=7.3). Eight fractions of 1 ml were collected and the IgG biotin concentration was determined by the absorbance at 280 nm.

The ELISA plates were prepared on EIA/RIA 1x8 Stripwell plate by adding 200 µl/well of GNA lectin at a concentration of 50 µg/ml in Ca²⁺/Mg²⁺ free PBS. Plates were wrapped in plastic foil and incubated overnight at +20°C. Wells were then aspirated and washed 4 times with 350 µl/well of wash buffer (PBS Ca²⁺/Mg²⁺ 1x - Tween 20 0.05%, pH 7.4). Then plates were incubated with 350µl/well of blocking buffer (Tris-HCl 10 mM - NaCl 150 mM - Sucrose 3% - BSA 1%, pH 7.68) for 1 hour at room temperature. Blocking buffer was aspirated and the plates could be immediately used for the assay or dried overnight at room temperature in a lyophilizer and then stored at +4°C in a heated sealed foil pouch with a desiccant pack.

For performing the sandwich ELISA seven 2-fold serial dilutions of the samples were made in Ca²⁺/Mg²⁺-free PBS. Each dilution was loaded in duplicates on GNA-coated well (100 µl/well) and incubated 30 minutes at 37°C with shaking. After the incubation, biotinylated-IgGs (diluted at 8 µg/ml in biotin diluent (50mM Tris - 150mM NaCl - 0.05% Tween20 - 20% lamb serum, pH 7.5) were added (100 µl/well) to the antigen without washing and incubated for other 30 minutes at 37°C with shaking. Wells were then aspirated and washed 4 times with 350 µl/well of wash buffer (50mM Tris - 150 mM NaCl - 0.05% Tween 20) with an automatic plate washer. IgG binding was then detected by adding 200 µl/well of streptavidin-conjugated alkaline phosphatase diluted at 1 µg/ml in wash buffer, for 30 minutes at 37°C with shaking. At the end of this step the plate was aspirated and washed as before, then 200 µl/well of pNPP was added to the wells and incubated 30 minutes at room temperature. Plates were read at 405nm in a microplate reader.

### ELISA specificity

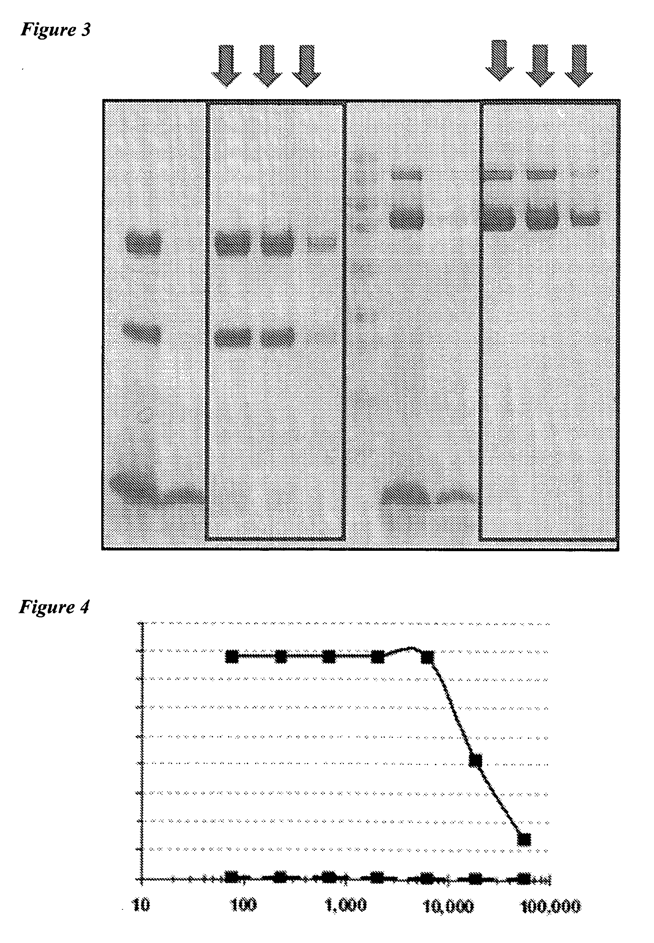

To confirm strain specificity the ELISA was tested using captured B/Florida/4/2006 antigen prepared from with IgG either for that strain or for the unrelated A/Solomon/3/2006 strain. Figure 4 shows OD₄₀₅ₙₘ for monovalent vaccine bulk prepared from MDCK cells (Optaflu bulk) against antigen dilution. The anti-B/Florida IgG reliably detected the B/Florida HA (solid line) but the anti-Solomon IgG did not (dotted line).

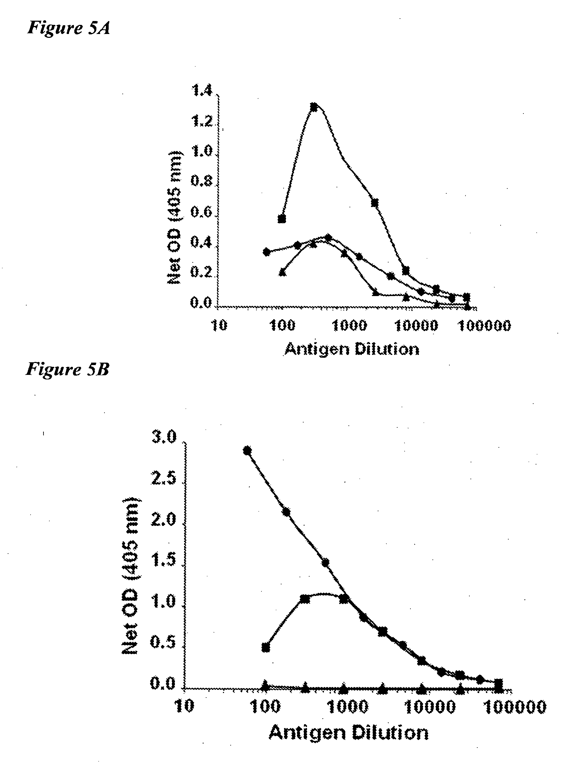

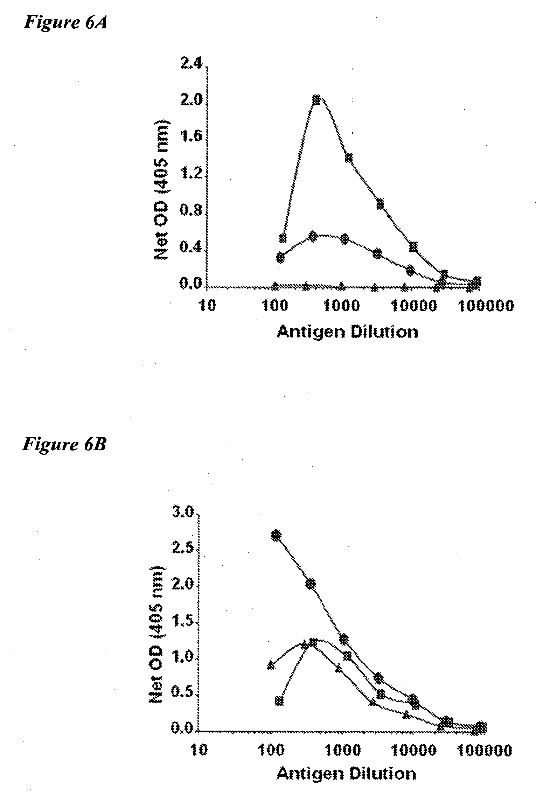

Similarly, IgG against A/Brisbane/59/07 (H1N1) or Uruguay/716/2007 (H3N2) were tested against the relevant reference antigens from NIBSC, and against monovalent Optaflu bulks for these strains. Figures 5 and 6 confirm specificity of the ELISA. H1N1 or H3N2 MDCK-produced monobulks were detected only by the IgG specific for each strain; the egg-produced CBER or NIBSC antigen standards generated positive ELISA signals with either the H1N1- or H3N2-specific antisera.

The most likely explanation for the cross-reactivity observed when sheep antisera raised against egg-produced HA were used to detect egg-produced antigen samples (but not when used to detect MDCK cell-produced antigen samples) is the presence of egg-derived proteins in the egg-produced antigen samples and anti-egg protein antibodies in the sheep antisera. This conclusion is corroborated by western blots (not shown) which indicate the cross reactive antibody recognizes proteins that do not co-migrate with HA.

### Broad strain applicability

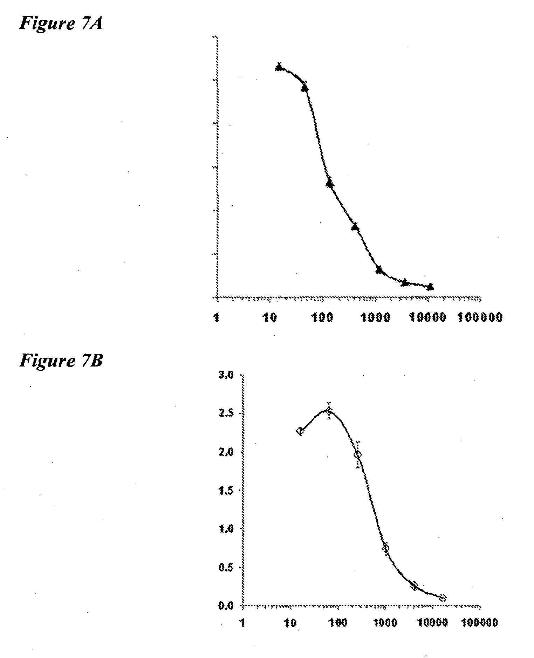

To confirm that the ELISA method is broadly suitable for influenza virus strains the GNA lectin capture was tested with monovalent bulk vaccine prepared from strains having low glycosylation, namely A/Vietnam/1194/2004 (H5N1) and A/California/04/2009 (H1N1). Figure 7 shows that the capture works well even with these strains. Thus the capture lectin is easily adaptable to different strains, independent of their HA glycosylation levels.

### Comparison with SRID

Samples were taken at 13 stages during production of a monovalent A/Brisbane/59/07 (H1N1) bulk vaccine. These were analysed for HA content by SRID, by RP-HPLC, or by the ELISA of the invention. The SRID and RP-HPLC assays were calibrated using the relevant NIBSC standard; the ELISA was calibrated either with purified A/Brisbane/59/07 hemagglutinin or with SRID-calibrated A/Brisbane/59/07 monovalent bulk. HA concentration in the ELISA was calculated as the mean of at least three different dilutions (coefficient of variation≤20%).

Results at each stage were as follows (µg/ml; '-' = not detectable):

| **Stage** | **SRID** | **RP-HPLC** | **ELISA¹** | **ELISA²** |
|---|---|---|---|---|
| 1 | - | 34 | 19 | 19 |
| 2 | - | 33 | 16 | 16 |
| 3 | - | 32 | 16 | 16 |
| 4 | - | 31 | 18 | 19 |
| 5 | - | 3 | 2 | 2 |
| 6 | - | 2 | - | - |
| 7 | - | 2 | 4 | 4 |
| 8 | - | 2 | - | - |
| 9 | 342 | 313 | 280 | 328 |
| 10 | 124 | 138 | 97 | 125 |
| 11 | 180 | 175 | 142 | 176 |
| 12 | 129 | 127 | 106 | 130 |
| 13 | 29 | 19 | 36 | 36 |

| | | | | |
|---|---|---|---|---|
| 1: ELISA reference was purified protein; 2: ELISA reference was SRID-calibrated monobulk | | | | |

Thus the ELISA was able to quantify HA even at levels too low for detection by SRID, and the quantitative results are in line with those measured by other techniques.

### Preparative HA purification

An affinity support with immobilised GNA lectin was obtained from Vector Laboratories Inc in Burlingame California (catalog #AL-1243), and was used with a GE Healthcare HR 16/10 (20ml) column. Monovalent bulk influenza vaccines were mixed with n-Octyl-beta-D-glucopyranoside (octylglucoside, OG; final concentration 1.5%) and incubated on ice. This material was applied to a washed column, and then eluted using α-methyl-mannopyranoside.

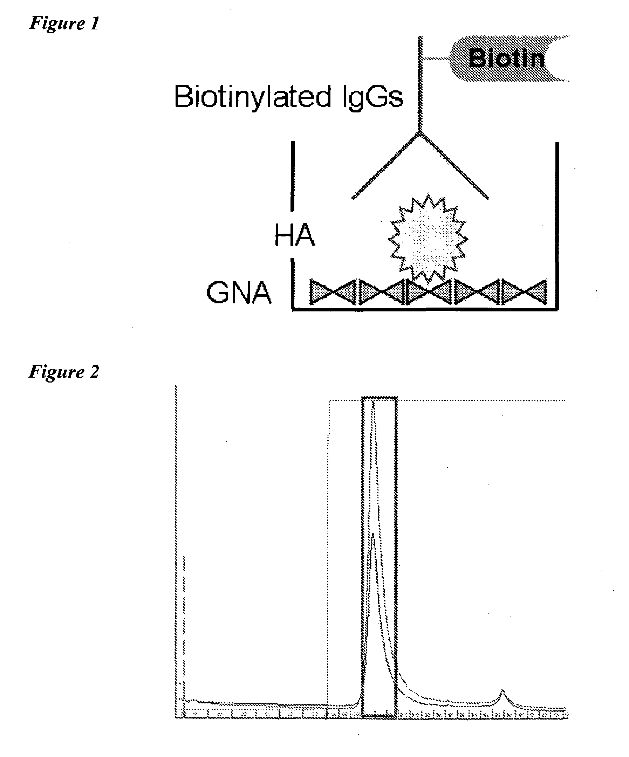

Figure 2 shows a chromatogram of material purified by affinity chromatography using immobilised GNA lectin with A/Brisbane/59/07 vaccine. The boxed fractions were pooled and dialysed against PBS. The dialysed material was analysed by SDS-PAGE (Figure 3). The HA is very pure and so GNA lectin affinity chromatography can be used for preparative purification of HA.

### Antigen standards for the HA ELISA

Figure 8 shows that when an NIBSC antigen standard and antiserum (both egg-platform based) are used to calibrate the ELISA format of the invention for detection of HA in an MDCK-produced vaccine monobulk, the HA content of the monobulk is grossly underestimated (27 µg/ml, first column from the left) relative to the content determined using the same reagents in SRID (365 µg/ml, fourth column from the left). Thus, it appears that the relative contributions of anti-HA and anti-host (egg) antibody-antigen interactions to the signal in ELISA and SRID are different. The inventors consider that, because the precipitin ring in SRID results from the antibody interactions of primarily the most abundant antigen in the sample (HA), host cell protein and anti-host cell protein antibody effects may be much less important in SRID than in ELISA.

The inventors hypothesized that using purified HA as an antigen standard would eliminate host cell protein effects on the ELISA, bringing its results into alignment with those obtained by SRID. To test this hypothesis, the inventors used HA produced in MDCK cells, purified on a GNA-column, and quantified by a conventional SRID at 109 µg/ml (Figure 8, first column from the right) using egg-based antigen standard and antiserum as the standard for the ELISA. The quantity of the purified HA determined by conventional SRID corresponded well to the quantity of HA in this sample determined by physical methods. Absorption at 280 nm indicated 100 µg/ml HA and BCA assay indicated 83 µg/ml HA. Using this purified HA standard brought the ELISA value for the MDCK-produced monobulk (327 µg/ml, second column from the left in Figure 8) into reasonable correspondence with the SRID value for the monobulk (365 µg/ml).

The hypothesis that matrix effects in the ELISA, but not in SRID, are responsible for discrepancies between ELISA and SRID predicts that an MDCK-produced monobulk, quantified by SRID with egg-based antigen standard and antiserum could be used to calibrate the ELISA for use with MDCK-produced samples, provided that the sheep antiserum used for detection in the ELISA was elicited by immunization with egg-produced HA. Consistent with this hypothesis, the quantity of MDCK-produced purified HA by ELISA (100 µg/ml, third column from the left in Figure 8) calibrated with this monobulk standard matched the quantity of the same purified HA sample, determined by standard SRID (109 µg/ml). Therefore, the data suggest that cell derived monobulk quantified with standard SRID-assigned HA concentrations can be used as standard for ELISA to quantify MDCK-produced antigens.

In conclusion, the ELISA is sensitive to anti-host (for example, egg) antibodies in conventional antisera used for SRID. Therefore, the data suggest that it is best to use serum raised against egg-produced HA to detect mammalian cell-produced HA. Antigen calibration standards for the ELISA format of the invention should be purified or at least should not be produced from the same platform that produced the antigen used to immunize the sheep to avoid such anti-host cell protein effects.

It will be understood that the invention has been described by way of example only and modifications may be made whilst remaining within the scope of the invention.

### REFERENCES

[1] Williams (1993) Vet Microbiol 37:253-262.
[2] Fitzgerald & Needy (1986) Dev Biol Stand 64:73-79.
[3] WO2010/136896.
[4] WO2005/090390.
[5] Li et al. (2010) Biologicals 38:284-9.
[6] Brady & Griffiths (1982) J Biol Standardization 10:9-15.
[8] Li et al. (2009) Curr Chem Viol 3:324-33.
[9] Wolff and Reichl (2011) Expert Rev. Vaccines 10:1451-1475.
[10] WO2007/066231.
[11] Opitz et al. (2007) Vaccine 939-947
[12] Optiz et al. (2008) Journal of Virological Methods 154:61-68
[13] Opitz et al. (2007) Journal of Biotechnology 131:309-317
[14] WO96/37624.
[15] WO98/46262.
[16] WO95/18861.
[17] Bright et al. (2008) PLoS ONE 3:e1501.
[18] Crevar & Ross (2008) Virology Journal 5:131.
[19] Vaccines. (eds. Plotkin & Orenstein). 4th edition, 2004, ISBN: 0-7216-9688-0.
[20] Huckriede et al. (2003) Methods Enzymol 373:74-91.
[21] GenBank sequence GI:325176.
[22] WO2008/032219
[23] WO97/37000.
[24] Brands et al. (1999) Dev Biol Stand 98:93-100.
[25] Halperin et al. (2002) Vaccine 20:1240-7.
[26] Tree et al. (2001) Vaccine 19:3444-50.
[27] EP-A-1260581 (WO01/64846).
[28] WO2006/071563.
[29] WO2005/113758.
[30] WO2008/001221.
[31] WO90/14837.
[32] Podda & Del Giudice (2003) Expert Rev Vaccines 2:197-203.
[33] Podda (2001) Vaccine 19: 2673-2680.
*[34]* Vaccine Design: The Subunit and Adjuvant Approach (eds. Powell & Newman) Plenum Press 1995 (ISBN 0-306-44867-X).
[35] Vaccine Adjuvants: Preparation Methods and Research Protocols (Volume 42 of Methods in Molecular Medicine series). ISBN: 1-59259-083-7. Ed. O'Hagan.
[36] WO2008/043774.
[37] Allison & Byars (1992) Res Immunol 143:519-25.
[38] Hariharan et al. (1995) Cancer Res 55:3486-9.
[39] US-2007/014805.
[40] WO95/11700.
[41] US patent 6,080,725.
[42] WO2006/113373.
[43] WO2005/097181.
[44] WO01/22992.
[45] Hehme et al. (2004) Virus Res. 103(1-2):163-71.
[46] Treanor et al. (1996) J Infect Dis 173:1467-70.
[47] Keitel et al. (1996) Clin Diagn Lab Immunol 3:507-10.
[48] Zangwill et al. (2008) J Infect Dis. 197(4):580-3.

## Claims

1. A method for analysing a vaccine comprising steps of:
(a) detecting influenza virus hemagglutinin in the vaccine, or a sample thereof, comprising steps of: (i) contacting the sample with a lectin which is immobilised on a solid support, to provide captured hemagglutinin; and (ii) detecting the captured hemagglutinin by immunoassay; and
(b) using the results of step (a) to calculate the hemagglutinin concentration in the vaccine.

2. The method of claim 1, wherein the lectin is a GNA lectin.

3. The method of any preceding claim, wherein step (ii) is a quantitative step which permits determination of the amount and/or concentration of hemagglutinin in the sample.

4. The method of any preceding claim, wherein the sample includes no viral proteins except for influenza virus proteins.

5. The method of any preceding claim, wherein the sample includes detergent.

6. The method of any preceding claim, wherein the sample is a bulk vaccine.

7. The method of any one of claims 1-6, wherein the hemagglutinin is quantified in an ELISA method comprising steps of: (i) contacting the sample with a lectin which is immobilised on a solid support, to provide captured hemagglutinin; (ii) attaching an enzyme to the captured hemagglutinin via an anti-hemagglutinin antibody; and (iii) using the attached enzyme to convert a substrate to a product, wherein quantitative detection of the product indicates the quantity of influenza virus hemagglutinin in the sample.

8. A method for providing a bulk vaccine with a desired hemagglutinin concentration, comprising steps of: (a) analysing hemagglutinin in the vaccine, or in a sample thereof, by the method of any one of claims 1-7; (b) using the results of step (a) to calculate the hemagglutinin concentration in the bulk vaccine; and (c) using the results of step (b) to dilute the bulk vaccine to give the desired hemagglutinin concentration.

9. The method of claim 8, wherein the desired hemagglutinin concentration is between 1 and 150µg/ml *e.g*. 90µg/ml, 45µg/ml, 30 µg/ml, 15 µg/ml, 10 µg/ml, 7.5 µg/ml, 5 µg/ml, 3.8 µg/ml, 3.75 µg/ml, 1.9 µg/ml, or 1.5 µg/ml.

10. A method for providing a vaccine for patient use, comprising steps of: preparing a bulk vaccine with a desired hemagglutinin concentration by the method of claim 8 or claim 9; and then extracting one or more unit doses of vaccine from the diluted bulk.

11. The method of claim 10, wherein an extracted unit dose is packaged as a kit component in combination with a second kit component, wherein the second kit component is a vaccine adjuvant.

12. A method for providing a bulk adjuvanted vaccine comprising steps of: preparing a bulk vaccine with a desired hemagglutinin content by the method of claim 8 or claim 9; and then mixing the diluted bulk vaccine with an adjuvant.

13. A method for providing an adjuvanted vaccine for patient use, comprising steps of: providing a bulk adjuvanted vaccine by the method of claim 12; and then extracting one or more unit doses of vaccine from the bulk.

14. A method for purifying influenza virus hemagglutinin in a sample, comprising a step of contacting the sample with a lectin which is immobilised on a solid support, wherein the lectin is a mannose-binding lectin, with the proviso that the lectin is not a ConA lectin.

15. The method of claim 14, wherein the lectin is a GNA lectin.

## Patentansprüche

1. Verfahren zum Analysieren eines Impfstoffs, das die folgenden Schritte umfasst:
(a) Nachweisen von Influenzavirus-Hämagglutinin in dem Impfstoff oder einer Probe davon, umfassend die Schritte: (i) Inkontaktbringen der Probe mit einem Lectin, das auf einem festen Träger immobilisiert ist, um eingefangenes Hämagglutinin bereitzustellen; und (ii) Nachweisen des eingefangenen Hämagglutinins mittels Immunassay; und
(b) Verwenden der Ergebnisse von Schritt (a) zum Berechnen der Hämagglutininkonzentration in dem Impfstoff.

2. Verfahren nach Anspruch 1, wobei es sich bei dem Lectin um ein GNA-Lectin handelt.

3. Verfahren nach einem vorhergehenden Anspruch, wobei Schritt (ii) ein quantitativer Schritt ist, der die Bestimmung der Menge und/oder Konzentration an Hämagglutinin in der Probe gestattet.

4. Verfahren nach einem vorhergehenden Anspruch, wobei die Probe außer Influenzavirus-Proteinen keine Virusproteine beinhaltet.

5. Verfahren nach einem vorhergehenden Anspruch, wobei die Probe Detergens beinhaltet.

6. Verfahren nach einem vorhergehenden Anspruch, wobei die Probe ein Bulk-Impfstoff ist.

7. Verfahren nach einem der Ansprüche 1-6, wobei das Hämagglutinin in einem ELISA-Verfahren quantifiziert wird, dass die folgenden Schritte umfasst: (i) Inkontaktbringen der Probe mit einem Lectin, das auf einem festen Träger immobilisiert ist, um eingefangenes Hämagglutinin bereitzustellen; (ii) Anheften eines Enzyms an das eingefangene Hämagglutinin mittels eines anti-Hämagglutinin-Antikörpers; und (iii) Verwenden des angehefteten Enzyms, um ein Substrat in ein Produkt umzuwandeln, wobei ein quantitativer Nachweis des Produkts die Menge des Influenzavirus-Hämagglutinins in der Probe anzeigt.

8. Verfahren zum Bereitstellen eines Bulk-Impfstoffs mit einer gewünschten Hämagglutininkonzentration, das die folgenden Schritte umfasst: (a) Analysieren von Hämagglutinin in dem Impfstoff oder in einer Probe davon nach dem Verfahren nach einem der Ansprüche 1-7; (b) Verwenden der Ergebnisse von Schritt (a) zum Berechnen der Hämagglutininkonzentration in dem Bulk-Impfstoff; und (c) Verwenden der Ergebnisse von Schritt (b) zum Verdünnen des Bulk-Impfstoffs, um zu der gewünschten Hämagglutininkonzentration zu gelangen.

9. Verfahren nach Anspruch 8, wobei die gewünschte Hämagglutininkonzentration zwischen 1 und 150 µg/ml, z.B. 90 µg/ml, 45 µg/ml, 30 µg/ml, 15 µg/ml, 10 µg/ml, 7,5 µg/ml, 5 µg/ml, 3,8 µg/ml, 3,75 µg/ml, 1,9 µg/ml oder 1,5 µg/ml liegt.

10. Verfahren zum Bereitstellen eines Impfstoffs zur Verwendung an Patienten, das die folgenden Schritte umfasst: Herstellen eines Bulk-Impfstoffs mit einer gewünschten Hämagglutininkonzentration nach dem Verfahren nach Anspruch 8 oder Anspruch 9; und anschließend Entnehmen von einer oder mehreren Impfstoff-Einzeldosen von dem verdünnten Bulk.

11. Verfahren nach Anspruch 10, wobei eine entnommene Einzeldosis als Kit-Bestandteil in Kombination mit einem zweiten Kit-Bestandteil verpackt ist, wobei es sich bei dem zweiten Kit-Bestandteil um ein Impfstoffadjuvans handelt.

12. Verfahren zur Bereitstellung eines Bulk-Impfstoffs mit Adjuvans, das die folgenden Schritte umfasst: Herstellen eines Bulk-Impfstoffs mit einem gewünschten Hämagglutiningehalt nach dem Verfahren nach Anspruch 8 oder Anspruch 9; und anschließend Mischen des verdünnten Bulk-Impfstoffs mit einem Adjuvans.

13. Verfahren zum Bereitstellen eines Impfstoffs mit Adjuvans zur Verwendung an Patienten, das die folgenden Schritte umfasst:
Bereitstellen eines Bulk-Impfstoffs mit Adjuvans nach den Verfahren nach Anspruch 12; und
anschließend Entnehmen von einer oder mehreren Impfstoff-Einzeldosen von dem Bulk.

14. Verfahren zum Aufreinigen von Influenzavirus-Hämagglutinin in einer Probe, das den Schritt des Inkontaktbringens der Probe mit einem Lectin, das auf einem festen Träger immobilisiert ist, umfasst, wobei es sich bei dem Lectin um ein mannosebindendes Lectin handelt, mit der Maßgabe, dass es sich bei dem Lectin nicht um ein ConA-Lectin handelt.

15. Verfahren nach Anspruch 14, wobei es sich bei dem Lectin um ein GNA-Lectin handelt.

## Revendications

1. Procédé d'analyse d'un vaccin comprenant les étapes de :
(a) détection de l'hémagglutinine du virus de la grippe dans le vaccin, ou un échantillon de celui-ci, comprenant les étapes de : (i) mise en contact de l'échantillon avec une lectine qui est immobilisée sur un support solide, pour obtenir une hémagglutinine capturée ; et (ii) détection de l'hémagglutinine capturée par immunodosage ; et
(b) utilisation des résultats de l'étape (a) pour calculer la concentration d'hémagglutinine dans le vaccin.

2. Procédé de la revendication 1, dans lequel la lectine est une lectine GNA.

3. Procédé de l'une quelconque des revendications précédentes, dans lequel l'étape (ii) est une étape quantitative qui permet la détermination de la quantité et/ou concentration d'hémagglutinine dans l'échantillon.

4. Procédé de l'une quelconque des revendications précédentes, dans lequel l'échantillon ne comprend aucune protéine virale à l'exception de protéines du virus de la grippe.

5. Procédé de l'une quelconque des revendications précédentes, dans lequel l'échantillon comprend un détergent.

6. Procédé de l'une quelconque des revendications précédentes, dans lequel l'échantillon est un vaccin en vrac.

7. Procédé de l'une quelconque des revendications 1 à 6, dans lequel l'hémagglutinine est quantifié dans un procédé ELISA comprenant les étapes de : (i) mise en contact de l'échantillon avec une lectine qui est immobilisée sur un support solide, pour obtenir une hémagglutinine capturée ; (ii) liaison d'une enzyme à l'hémagglutinine capturée par l'intermédiaire d'un anticorps anti-hémagglutinine ; et (iii) utilisation de l'enzyme liée pour convertir un substrat en produit, dans lequel la détection quantitative du produit indique la quantité d'hémagglutinine du virus de la grippe dans l'échantillon.

8. Procédé de production d'un vaccin en vrac ayant une concentration d'hémagglutinine souhaitée, comprenant les étapes de : (a) analyse de l'hémagglutinine dans le vaccin, ou dans un échantillon de celui-ci, par le procédé de l'une quelconque des revendications 1 à 7 ; (b) utilisation des résultats de l'étape (a) pour calculer la concentration d'hémagglutinine dans le vaccin en vrac ; et (c) utilisation des résultats de l'étape (b) pour diluer le vaccin en vrac pour obtenir la concentration d'hémagglutinine souhaitée.

9. Procédé de la revendication 8, dans lequel la concentration d'hémagglutinine souhaitée est comprise entre 1 et 150 µg/ml, par exemple, 90 µg/ml, 45 µg/ml, 30 µg/ml, 15 µg/ml, 10 µg/ml, 7,5 µg/ml, 5 µg/ml, 3,8 µg/ml, 3,75 µg/ml, 1,9 µg/ml ou 1,5 µg/ml.

10. Procédé de production d'un vaccin pour utilisation sur des patients, comprenant les étapes de : préparation d'un vaccin en vrac ayant une concentration d'hémagglutinine souhaitée par le procédé de la revendication 8 ou la revendication 9 ; et ensuite l'extraction d'une ou plusieurs doses unitaires de vaccin à partir du vrac dilué.

11. Procédé de la revendication 10, dans lequel une dose unitaire extraite est conditionnée sous la forme d'un composant de kit en combinaison avec un deuxième composant de kit, le deuxième composant de kit étant un adjuvant de vaccin.

12. Procédé de production d'un vaccin avec adjuvant en vrac comprenant les étapes de : préparation d'un vaccin en vrac ayant une teneur en hémagglutinine souhaitée par le procédé de la revendication 8 ou la revendication 9 ; et ensuite mélange du vaccin en vrac dilué avec un adjuvant.

13. Procédé de production d'un vaccin avec adjuvant pour utilisation sur des patients, comprenant les étapes de : production d'un vaccin avec adjuvant en vrac par le procédé de la revendication 12 ; et ensuite extraction d'une ou plusieurs doses unitaires de vaccin à partir du vrac.

14. Procédé de purification d'hémagglutinine de virus de la grippe dans un échantillon, comprenant une étape de mise en contact de l'échantillon avec une lectine qui est immobilisée sur un support solide, dans lequel la lectine est une lectine de liaison de mannose, à condition que la lectine ne soit pas une lectine ConA.

15. Procédé de la revendication 14, dans lequel la lectine est une lectine GNA.
